# EUROPEAN PATENT APPLICATION

(11) **EP 3 763 291 A1**
(43) Date of publication of application: **13.01.2021**
(21) Application number: 19763804.2
(22) Date of filing: 27.02.2019
(51) Int. Cl.: A61B 5/145, A61B 5/00

(54) **BLOOD GLUCOSE MONITORING METHOD AND WEARABLE BLOOD GLUCOSE MONITORING DEVICE USING SAME**

(30) Priority: 05.03.2018 KR 20180025954
(71) Applicant: Biomedilabs Co., Ltd., Seoul 06542 (KR)
(72) Inventor: KIM, Doug Won, Seoul 01690 (KR)
(74) Representative: Lichti - Patentanwälte Partnerschaft mbB
(86) International application number: PCT/KR2019/002352
(87) International publication number: WO 2019/172570

(57) **Abstract**

Disclosed are a blood glucose monitoring method and a PPG-based blood glucose monitoring device for same in which a reference sample is prepared by means of preparing a human body substitute dummy and reference blood from which blood glucose is removed and applying quasi-blood to the dummy, the signal amount of each wavelength band with respect to the reference sample to which the reference blood is applied is measured by means of a blood glucose monitoring device, reference signal amount data is obtained and provided as basic data, irradiation light of a plurality of light sources is radiated on a body part of a subject for blood glucose monitoring by means of the blood glucose monitoring device of the present invention, a light-receiving element receives reflected light and scattered light and thus the signal amount of each wavelength band is obtained, a differential signal amount for each wavelength band, which is a corresponding reference signal amount for each wavelength band comprised in the basic data subtracted from the signal amount for each wavelength band with respect to the body part of the subject for blood glucose monitoring, is obtained, and a blood glucose amount corresponding to the differential signal amount for each wavelength band is calculated by means of the correlation between the differential signal amount for each wavelength band and the blood glucose amount.

## Description

### Technical Field:

The present invention relates to a blood glucose monitoring method and a wearable blood glucose monitoring device using the same, and more particularly, to a noninvasive blood glucose monitoring method and a wearable blood glucose monitoring device having a small-sized simple structure using the same.

### Background Art:

In order to measure blood glucose, in general, there is a blood collection type glucose monitoring method for collecting some of blood and measuring blood glucose using the collected blood. However, such a blood collection type glucose monitoring method may inflict pain to a subject to be measured, and it takes much time and it is complicated since measurement is carried out after collecting blood.

In order to solve the above problem, recently, studies and developments on technology to measure blood glucose without collecting blood have been achieved.

As the blood collection type glucose monitoring method, there is a blood glucose monitoring method using closed spectroscopy. A noninvasive glucose monitoring device using the spectroscopy includes a light source, a sensor, and an analyzer for analyzing a result sensed by the sensor to show a numerical value of blood glucose. When infrared light from the light source penetrates a human body, the sensor senses it and converts it into an electric signal, and may be mounted on the human body using a pressure cuff.

Here, blood glucose is measured by using scattering of light. If a finger is put into the cuff, a round space gets narrower, and the cuff applies pressure of more than blood pressure of the contraction phase to the finger for two to three seconds. A blood flow of the finger is closed, and in this instance, an infrared light source projects infrared light to the blood flow closed part of the finger of a patient. When the blood flow of the finger is closed, various particles causing scattering of infrared light are condensed, and scattering of infrared light is reduced since the number of that the infrared light collides against the particles is reduced differently from the case that various particles are separated from one another before the blood flow is closed.

The infrared light reduced in scattering collides against more glucoses in a blood vessel. The glucose reduces a refractive index between the particles causing scattering of light and blood plasma surrounding the particles and reduces the quantity of photons absorbed into the blood vessel so that intensity of infrared light penetrating the blood vessel increases.

The noninvasive blood glucose monitoring device utilizing closed spectroscopy senses intensity of penetrating infrared light, analyzes it by an analyzer, and converts it into a numerical value of blood glucose using that infrared light entering the blood vessel is changed in scattered level according to concentration of glucose contained in blood so that intensity of the penetrating infrared light is varied.

However, besides blood glucose, there are many factors influencing on penetration of infrared light during noninvasive blood glucose measurement, and sometimes it is difficult to accurately measure blood glucose because of doses of medicine, etc.

Besides the noninvasive blood glucose monitoring method, there are Raman spectroscopy, optical coherence tomography, polarimetry, photoacoustic spectroscopy, and so on. The above methods are fully noninvasive methods and have no stimulation since it measures blood glucose concentration using light, but may have various limitations in enhancing accuracy. So, in order to overcome the above problem, various technologies are being studied.

Onsens Company developed a photonic glucose sensor (PGS) system using near infrared light. The PGS system includes a main sensor, two wrist bands, and a battery, and can measure blood glucose during a user's sleep since being continuously used for about 30 minutes. Moreover, the system transfers the measurement result data to the user's smart phone in real time through Bluetooth. The sensor system sends near infrared light into the skin at 5-minute intervals and analyzes blood glucose using information of light reflected. When infrared light is reflected at a narrow angle, the sensor system determines that an amount of glucoses in the skin is small, and when infrared light is reflected at a wide angle, the sensor system determines the amount of glucose in the skin is big.

However, the optical device and sensor system occupies a wide space in arrangement, and it is not easy that the user acts after mounting the sensor system on his or her wrist in a band type. Therefore, the sensor system has inconvenience in use.

Korean Patent No. 0300960 discloses noninvasive monitoring method and device for measuring blood component concentration. The noninvasive monitoring method and device backwardly scatters or penetrates light from or to an affected part using multicolored light sources emitting specific light in 800 nm to 1850 nm near infrared light regions. Because the light back-scattered from a tissue and a blood vessel containing blood has information on blood component concentration, the device properly receives light in order to avoid surface reflection on the surface of the skin and minimize an influence related with a change in back-scattering background. The blood component concentration is calculated by a spectroscopic analysis on the basis of a wavelength selected by a proposed algorithm. A micro-processor calculates a predetermined rate in response to a measurement curve stored in a memory of the microprocessor and determines blood component concentration, and the concentration value is displayed on a display.

However, in order to differentiate infrared energy absorption by blood glucose from infrared energy absorptions by other components and induce the absorption level of near infrared energy absorbed by blood glucose, the blood glucose monitoring device measures blood glucose concentration by correction induced by multivariate statistics of partial least square (PLS) relative to the components. Such a correction method is very complicated since requiring quantitative measurement data relative to all skin components, and also has a disadvantage in that it does not consider a near infrared signal transition by the user's skin characteristics.

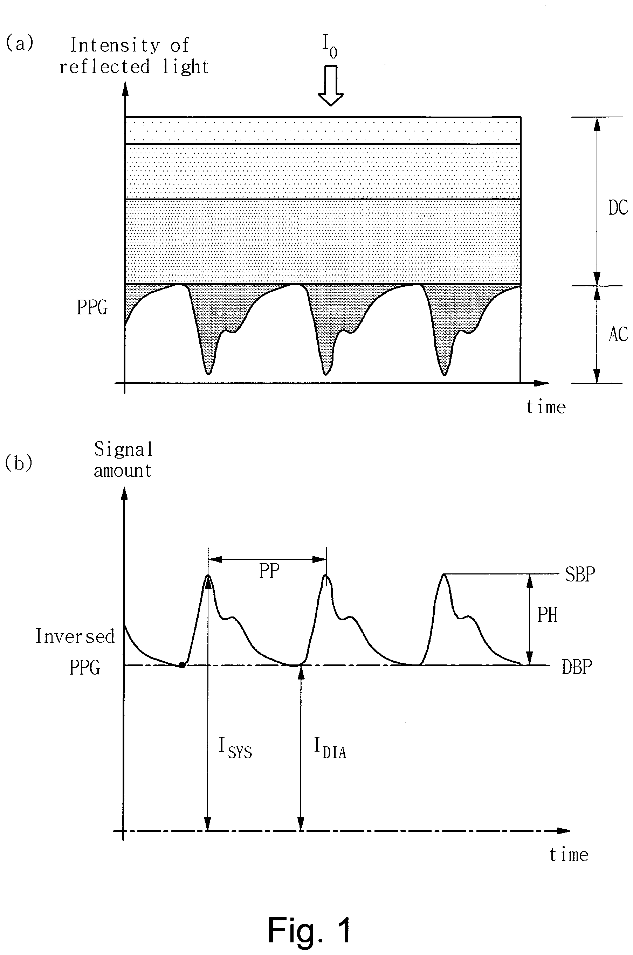

FIG. 1 shows a general form of pulse waves measurable by the PPG on the wrist and an AC component form which is a pure blood flow change that a DC component is excluded from the general form of the pulse wave.

The PPG is to analyze a pulse wave form at the early stage and measure a heart rate (HR) from a change in a blood flow rate, but recently, the AC component and the DC component showing the change in pulse wave can be separately reanalyzed using digital signal process (DSP) technology.

While using the PPG, the AC component is a part influenced by a pulse component of arterial blood, a pulse frequency may be used as a fundamental material to measure blood pressure (BP) and can obtain information of other body conditions, for instance, vascular aging information from a second derivative waveform (SDPPG) of the AC component. The DC component is mostly influenced by skin and tissues, venous blood, non pulsatile component of arterial blood, and so on.

A photosensor assembly of the body wearable PPG generally uses green, red, and near infrared (NIR) LEDs as light sources and measures a change in reflected light intensity depending on the blood flow rate using a photodiode (PD), and the reflected light intensity is in an inverse relationship to an absorption coefficient.

The PPG includes a light source for shining light towards the blood vessels of a human body and a light receiving device like a photodiode for measuring intensity of reflected or scattered light, and blood pressure rises or a great deal of light is absorbed by blood at the peak of the contraction phase where the blood flow rate by contraction of the heart increases. In FIG. 1(a), the solid line of the upper part indicates intensity (I₀) of incident light, and the lowermost dashed solid line indicates intensity of reflected light. For intuitive cognition, the PPG is configured to show a high output signal when blood pressure is high, and a value that intensity of the reflected light is subtracted from intensity of incident light is indicated in the form of a signal amount of FIG. 1(b) (not an integral type signal amount). That is, an output signal in the general PPG is not intensity of the reflected light but comes out in the form of an inversed PPG, so that the blood flow rate or the blood pressure level are shown as a high peak in the contraction phase and as a low valley in the relaxation phase in the form of a pulse wave.

In the meantime, because blood pressure is not 0 even in the relaxation phase, a periodic amplitude change of the AC component on the base level is made.

### Disclosure:

### Technical Problem:

Accordingly, the present invention has been made in an effort to solve the above-mentioned problems occurring in the conventional noninvasive blood glucose monitoring methods, and it is an object of the present invention to provide a blood glucose monitoring method and a wearable blood glucose monitoring device using the same, which can measure a blood glucose value accurately in a noninvasive manner.

It is another object of the present invention to provide a blood glucose monitoring method and a wearable blood glucose monitoring device using the same, which can continuously measure a blood glucose value by being easily worn on a user's body due to its simple structure and easily grasp a development and a changing tendency of a blood glucose numerical value.

### Technical Solution:

To achieve the above objects, the present invention provides a blood glucose monitoring device including: a plurality of light sources for projecting near infrared lights with different wavelengths to a subject to be measured; light receiving devices for receiving lights reflected and scattered from the subject after being projected from the plurality of light sources and for converting the received light into an electric signal; and an analyzing module for analyzing the electric signal of the photodiodes, obtaining a signal amount from a pulse wave type signal relative to each wavelength, and calculating blood glucose concentration in blood of the subject using a differential signal amount between the signal amount obtained relative to the body of the subject to be measured and a reference signal amount inputted previously.

The blood glucose analyzing device according to the present invention may be configured on the basis of the operational principle of the existing PPG.

The blood glucose analyzing device according to the present invention may further include a reference light source with a visible wavelength range which can project light of unreactive wavelength range which is not absorbed into the blood glucose in blood as well as the plurality of light sources with near infrared wavelength range, wherein the reference light source may project green light.

In the present invention, the signal amount may be an integral value relative to one cycle indicated by pulse wave when the electric signal outputted from the photodiode, which receives reflected and scattered light caused by the light projected from the light source is shown in the form of pulse wave according to a change in blood flow rate.

In the present invention, mutual ratios of the differential signal amounts by wavelength ranges are considered to be uniform relative to blood glucose, and the analyzing module is set to check and verify whether or not the mutual ratios corresponds to blood glucose when the differential signal amount by each wavelength range is obtained.

The light sources may emit neural light such as green light with 530 nm wavelength range, short near infrared light with 940 nm wavelength range, near infrared light with 1450 nm wavelength range, and near infrared light with 1700 nm wavelength range.

In another aspect of the present invention, to achieve the above objects, the present invention provides a blood glucose monitoring method including the steps of: preparing a human body substitute dummy and reference blood from which blood glucose is removed, and applying the reference blood to the dummy to prepare a reference sample; measuring a signal amount by wavelength range relative to the reference sample to which the reference blood is applied by the blood glucose monitoring device in order to obtain a reference signal amount data as basic material data; projecting lights of a plurality of light sources relative to a body part of a subject to be measured and receiving reflected light and scattered light by photodiodes to obtain a signal amount by wavelength range; subtracting the reference signal amount by the corresponding wavelength range included in the basic material data from the signal amount by wavelength range relative to the body part of the subject to be measured, and deducing a differential signal amount by the wavelength range; and calculating the blood glucose level corresponding to the differential signal amount by wavelength range using relationship between the differential signal amount by wavelength range and the blood glucose level.

The reference signal amount data is a reference signal amount which is obtained in the form of a reference curve relative to all wavelength ranges while the blood glucose monitoring device densely projects lights of plural wavelength ranges using the near infrared spectrometer and is stored in a memory in the analyzing module of the blood glucose monitoring device, or a reference signal amount which is obtained in the form of an individual reference signal amount set relative to lights of several main wavelength ranges and is stored in the analyzing module of the blood glucose monitoring device.

In this instance, a plurality of reference curves or individual reference signal amount sets by intensity of light may be obtained while intensity of light or the energy level of the light sources are varied, and then, may be stored as the basic material data of the blood glucose monitoring device.

Additionally, the blood glucose monitoring device may further include a green light source as the reference light source beside the plurality of near infrared light sources, the reference curve or the individual reference signal amount set that the reference signal amount relative to the light of the green light source becomes equal to the signal amount measured relative to the body part of the subject to be measured is selected from the reference curves or the individual reference signal amount sets obtain by intensity of projected light or by the energy level in order to calculate the differential signal amount by wavelength range.

After the step of calculating the differential signal amount by wavelength range, a step of inspecting the mutual ratios between the differential signal amounts by wavelength ranges and checking whether or not the mutual ratios accord with the signal amount ratios in the wavelength ranges relative to the blood glucose. Such a step is to enhance accuracy in measurement of blood glucose level and prevent disturbance in measurement of the blood glucose level by unexpected factors.

The step of preparing the reference sample by applying the reference blood, from which blood glucose is removed, to the human body substitute dummy and the step of calculating the signal amount by wavelength range relative to the reference sample by the blood glucose monitoring device and another accurate blood glucose monitoring device may be previously carried out before measuring to the subject to be measured, so that the reference data is stored in the analyzing module and is prepared in such a way as to be drawn out and carry out comparison in case of necessity.

### Advantageous Effects:

The blood glucose monitoring method and the blood glucose monitoring device according to the present invention can accurately measure blood glucose of the subject to be measured in a noninvasive manner by changing a digital signal processing method through a software control in the analyzing module and including the plurality of near infrared light sources varying in wavelength ranges in the PPG.

The blood glucose monitoring method and the blood glucose monitoring device according to the present invention can continuously measure blood glucose by being carried easily in a state where the blood glucose monitoring device is easily worn on a body part of the subject to be measured like a wrist band since having a simplified optical structure and reduced size.

Therefore, the blood glucose monitoring method and the blood glucose monitoring device according to the present invention can easily grasp a change of state through a continuous measurement, give warning to the wearer when abnormality in the change of state is detected, and report and warn to a management organization through communication if a communication module is included.

### Description of Drawings:

FIG. 1 is a graph showing a general photoplethysmographic change and a change of pure AC component by a photo-plethysmography.
FIG. 2 is a relationship graph showing a relationship between several chemical combinations in blood components and near infrared wavelength having a great reaction to the chemical combinations in an absorption spectrum form.
FIG. 3 is a graph showing an example of a reference curve showing a change in signal amount according to a wavelength change obtained by applying reference blood to a dummy.
FIG. 4 is a graph showing an example of a differential signal amount.
FIG. 5 is a conceptual diagram showing a schematic configuration of a blood glucose monitoring device according to an embodiment of the present invention.
FIG. 6 is a bottom side view showing the blood glucose monitoring device based on a photo-plethysmography according to the embodiment of the present invention.
FIG. 7 is a conceptually sectional view taken along the line AA' of FIG. 6.
FIG. 8 is a conceptual diagram showing an example of the blood glucose monitoring device having a different arrangement of light sources and a photodiode.
FIG. 9 is a flow chart showing a glucose monitoring method according to an embodiment of the present invention.

### Mode for Invention:

Hereinafter, reference will be now mentioned in detail to the preferred embodiments of the present invention with reference to the attached drawings.

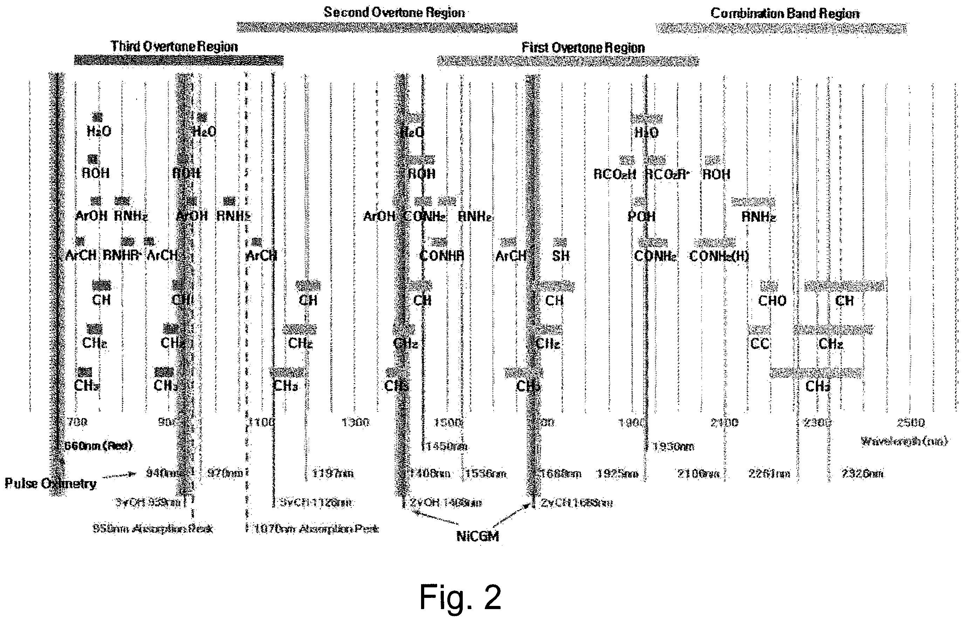

FIG. 2 is a relationship graph showing a relationship between several combinations in chemical materials and near infrared wavelength showing an absorption peak by greatly reacting to the chemical combinations in an absorption spectrum form.

Here, according to the drawing, it is shown that the absorption peak is strong relative to several wavelength ranges, such as red light with a 660 nm wavelength range, short near infrared light with a 940 nm wavelength range, near infrared light with a 140 nm wavelength range, and near infrared light with a 1688 nm wavelength range. Here, not shown in the drawings, when the red light with 660 nm wavelength is used, a strong light absorption by oxidized hemoglobin is generated, and when the short near-infrared light with 940 nm wavelength range is used, a strong light absorption by hemoglobin is generated so that we can find oxygen concentration or oxygen saturation of blood. Therefore, the concept that red light of 660 nm and a short near infrared light of 940 nm are used to carry out pulse oximetry and a light source with a single wavelength and a photodiode (PD) are used to measure a heart rate in a smart band or a smart watch is disclosed conventionally. And blood pressure and relevant characteristics are calculated additionally using the above relationship graph and it may be an index related with not only blood glucose but also pulse, blood pressure, and blood vessel.

Here, with respect to near-infrared rays with the above-mentioned wavelength range, an energy band which is easily absorbable by chemical combination of ROH, AROH, CH and so on is overlapped to the 940 nm wavelength range, an energy band which is easily absorbable by chemical combination of H₂O, ROH, ArOH, CH₂, CH₃ and so on is overlapped to the 940 nm wavelength range, and an energy band which is easily absorbable by chemical combination of CH, CH₂, CH₃ and so on is overlapped to the 1688 nm wavelength range.

The absorption level in the wavelength range of various materials including glucose has been widely known by the existing infrared spectroscopy, and mutual ratios of the absorption levels in the wavelength ranges are nearly uniform.

The present invention measures glucose concentration or the blood glucose level in blood using such infrared spectroscopic data. However, blood includes various materials including water, blood plasmas, and blood cells, and especially, if the PPG is used, because light getting out of the light source reaches blood after passing through the skin and other human tissues, the PPG performs infrared spectroscopy to the skin, the human tissues and the blood whenever blood glucose is measured. Therefore, in order to measure blood glucose, the basic data of the above materials must be considered, and it requires lots of efforts to measure blood glucose.

Therefore, when the present invention uses the infrared spectroscopy, for more simple analysis, the present invention does not absolutely analyze blood which is to be measured but carries out infrared spectroscopy to a subject (reference) of a base state on the basis of standard blood or reference blood from which blood glucose is removed so as to obtain a base data or a reference graph, and then, the data is previously inputted into an analyzing module.

Additionally, in the present invention, infrared spectroscopy is carried out to the subject's blood including blood glucose while keeping conditions except for blood glucose of the reference subject to be the most similar, so as to obtain a subject-related data or a subject-related graph.

Here, the present invention uses the PPG type device and projects infrared light to the human body and the photodiode receives and analyzes the light reflected and scattered from the human body in order to measure the blood glucose level of the subject. During measurement, instant reflection and intensity of scattered light are changed according to a blood flow rate, and the blood flow rate is changed depending on changes of pulse waves by time. However, because the blood glucose level is uniform even though the change of pulse waves rises, in order to obtain a stable value, the present invention does not use instant reflection and intensity of scattered light in measurement of the blood glucose level, but uses a signal amount as an integral value or an average time value obtained when an amount of instant signals, for instance, an amount of light absorption is integrated relative to one cycle on the premise that the pulse waves are stable and cyclic (hereinafter, the terms of "signal amount" means an integral value).

First, in order to measure the standard blood from which blood glucose is removed, a subject to be measured by a glucose monitoring device is prepared. However, if the glucose monitoring device is mounted on the wrist of a human body, near infrared light investigated from the glucose monitoring device passes a route including materials influencing on the DC component, such as the skin, cutaneous tissues. So, in order to measure blood glucose in a noninvasive manner, a value (signal amount) obtained in such a way that near infrared light is projected only to the standard blood and reflected and scattered light is received and measured cannot be used as the reference value.

Therefore, ideally, it is good to obtain a reference value (signal amount) by applying the standard blood to a target part of the human body to be measured. However, because it is not practically possible to apply the standard blood to the human body, a base is formed in such a way as to make a dummy having conditions similar to the target part of the human body to be measured and apply the standard blood to the dummy, and a reference value is obtained using the base. Such a dummy may be made using a pig's body part having little subcutaneous fat, which is very similar to the human body in components. The original blood is removed and the standard blood is injected into the blood vessel of the dummy so as to create the dummy similar to the blood vessel of the human body.

In order to measure the reference value by applying the infrared spectroscopy to the dummy, near infrared light including a near infrared band to be used first is projected to the dummy, the photodiode receives reflected and scattered light, and then, data on the size of the signal amount by wavelength bands is obtained and stored by an analyzing module. In this instance, since there is no change in a pulse wave form, the signal amount is obtained by an integral value which the uniform instant signal amount is simply multiplied by the pulse wave cycle (time).

The data may be expressed in the form of a numerical value of the signal amount relative to the plurality of light sources by wavelength bands to be measured by the PPG type glucose monitoring device, but preferably, may be expressed in a continuous graph curve which continues the numerical value of the signal amount relative to a continuous wave range including the wavelength band, or a subdivided wavelength band. In this instance, in order to measure the continuous wavelength band, an NIR spectrometer may be used.

The graph curve greatly depends on sensing characteristics (sensitivity by wavelength range) of the light receiving device, namely, the photodiode, and mutual ratios of the signal amounts by the wavelength ranges to be measured may not form a simple linear relationship like a simple proportional relationship. In this aspect, when the NIR spectrometer is used, it is preferable that the photodiode for measurement have the same characteristics as the photodiode of the glucose monitoring device.

In order to make a reference curve (graph curve), a plurality of reference curves are made by the size of the signal amount while adjusting the signal amount obtained through a method of controlling the quantity of light of the light source of the measuring device, such as the NIR spectrometer, and then, are used to acquire the blood glucose level. When the reference curve relative to two neighboring signal amounts is created, a reference curve between the two signal amounts may be made presumptively, and it is also possible to obtain the reference curve in all signal amount levels through the above work, which is known commonly. A statistic technique may be applied to the work, and deduction using an artificial neural network is also possible. Such a method may be achieved using an application program embedded in the analyzing module in the glucose monitoring device, so necessary base data may be stored in a memory of the blood glucose analyzing module.

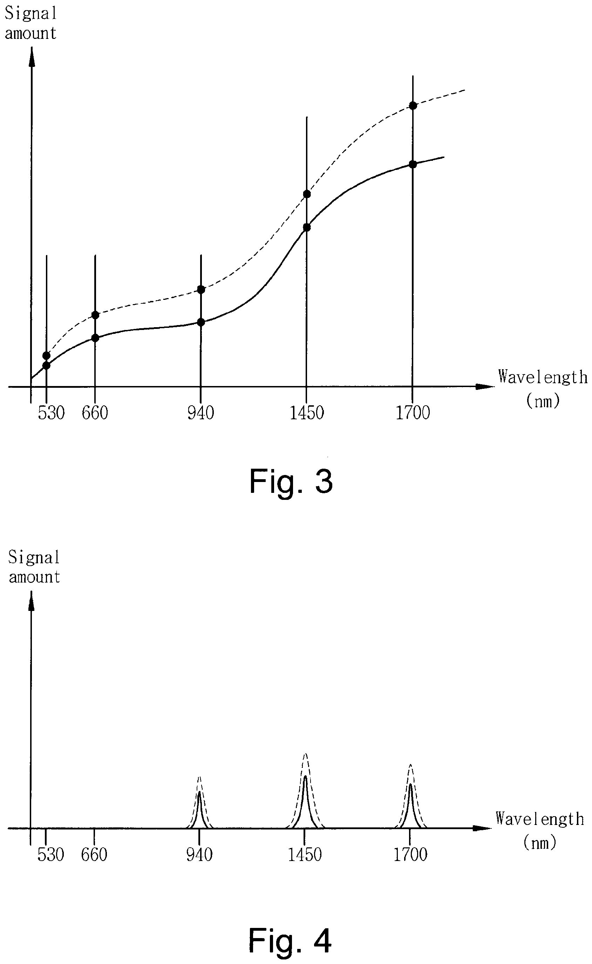

FIG. 3 is a view showing an example of such a reference curve.

The reference curve is the sum of the signal amounts in a near infrared area by various materials of the base, and peaks of the signal amounts by the materials are all involved therein. In order to make the reference curve accurate and delicate, besides the work to measure the signal amount while changing by applying near infrared light to the dummy in the near infrared band, work to check an aspect of the peak of a signal amount change curve according to wavelength of near infrared light relative to the previously known components to check and verify whether measurement is carried out correctly may be accomplished.

Of course, the reference value may be data of a discrete value set like the signal amount value (numerical value) by wavelength range to be measured as well as the reference curve. As described above, the reference value is inputted to the memory of the blood glucose analyzing module in various forms (the graph curve, the simple numerical data, or others).

When the obtained reference value is prepared as the standard for measurement, the body part of the subject to be measured is measured. For measurement, the glucose monitoring device of the present invention is used, a signal amount by each wavelength range related with the plurality of embedded light sources is obtained by the glucose monitoring device.

Moreover, a reference signal amount is subtracted from the signal amount of the subject to be measured by each wavelength range in order to calculate and obtain a differential signal amount by wavelength range. A result of the calculation may be expressed into a peak graph like the form shown in FIG. 4.

In this instance, ideally, the signal amount forming the reference value in the wavelength range which does not relate to blood glucose is exactly the same as the signal amount measured relative to the subject to be measured and has the value of 0.

However, due to differences between the dummy and the human body in quantitative and qualitative aspects, it is difficult that the differential signal value relative to the reference light source becomes zero even though the signal value is obtained in the same measurement condition. The reference and the measuring device relative to the subject to be measured have sameness qualitatively, but if there is a difference in scale, the intensity of radiation of the light source, the intensity of the photodiode, and other may have an influence on the size of the signal amount.

Therefore, in order to obtain the differential signal amount, the signal value obtained in the same measurement condition, for instance, under the condition that the same output is applied to the light sources, with respect to the reference subject (dummy) and the subject to be measured is not used, the reference curve or the reference signal amount set having the same signal amount as the signal amount obtain as the result that the subject to be measured is measured by the glucose monitoring device of the present invention in the wavelength range of the reference light is selected so as to obtain the differential signal amount in each wavelength range related with the plurality of light sources of the glucose monitoring device, and the differential signal amount is used to calculate the blood glucose amount.

In other words, because it ignores a physical difference or a base difference between the dummy and the subject to be measured to detect the signal mounts by wavelength ranges of the dummy and the subject to be measured under the same condition using the glucose monitoring device of the present invention, derive the differential signal amounts by wavelength ranges, and calculate the blood glucose amount under the assumption that the values are all related with the blood glucose amount, there is a problem. In this instance, it is required to match the level of the signal amount using light of the wavelength range which is not related with blood glucose.

For this, in the glucose monitoring device of the present invention, a light source, for instance, a green light source, for generating light of a visible light area, in which light is easily absorbed by the base part of the human body, together with the plurality of light sources for generating lights of different near infrared areas, is mounted on a first optical sensor assembly as a reference light source. In this instance, as conceptually mentioned above, it may be considered that the base part comprehensively expresses a part influencing on the size of the signal amount as well as blood glucose.

The green light does not express particular absorption level with respect to chemical combination related with blood glucose. However, because a range of fluctuation of a reflection level or an absorption level by the materials forming the base is great, the green light may be a reference to recognize the fluctuation in signal amount by the base and correct the signal amount in each wavelength range.

For example, when a reference signal amount data in the form of the reference curve (a continuous form) or the discrete reference signal amount set (discontinuous specific values) by output of the light source while varying light intensity of the plurality of light sources or the output level applied to the light sources, reference signal amounts by output levels relative to green light are obtained. Furthermore, the measuring device obtains also a signal amount value relative to the body part of the subject to be measured by green light in order to find a reference signal amount which is on par with the signal amount of the subject to be measured. Next, the measuring device find an output level for expressing the reference signal amount, and finds the reference signal amount set by wavelength range by the plurality of light sources when such an output level is applied. Additionally, the blood glucose measuring device obtains a differential signal amount set compared with the signal amount set by wavelength range measured relative to the body part of the subject to be measured.

Adjustment (selection) of the reference signal amounts by wavelength range can be carried out by a process of deducting reference signal amounts relative to green light when the above-mentioned base data is deducted, and then, adopting the reference signal amounts by wavelength range related with a proper reference signal amount relative to green light by using the obtained base data.

Of course, such a process may be automatically carried out through the application program embedded in the blood glucose analyzing module.

After the differential signal amount by each wavelength range is obtained through the above process, blood glucose of the subject to be measured can be calculated using the size of the differential signal amount. For this, as preliminary work, an associative relationship of the differential signal amount according to changes in blood glucose amount may be obtained through a clinical test as one of base data.

That is, if the differential signal amounts relative to blood with various blood glucose amounts are obtained and the associative relationship between the blood glucose amounts and the differential signal amounts are obtained by using multiple regression analysis or artificial neural network to obtain the differential signal amounts, the analyzing module can directly calculate the blood glucose amount.

In this instance, in order to calculate the blood glucose amount, it may be in question to select which one in the differential signal amounts to wavelength ranges. Ideally, the same blood glucose amount must be acquired even though the differential signal amount is selected in any wavelength range.

However, there is possibility that the blood glucose amounts obtained by the differential signal amounts by wavelength ranges are different from each other because the signal amount value measured relative to the subject to be measured is wrong from the start or an element besides blood glucose enters not to be suitable for measurement of the blood glucose amount. In this instance, it is difficult to calculate the blood glucose amount accurately.

Therefore, in order to measure the blood glucose amount accurately, the present invention further includes a step of verifying a mutual ratio between the differential signal amounts by wavelength ranges before calculating the blood glucose amount.

Because various ratio verifications can be achieved when there are many light sources for projectiong light of different near infrared wavelength ranges, it is more likely to secure accurate measurement of the blood glucose amount through intercomparison. However, in consideration of cost-effectiveness of manufacturing the devices, preferably, the number of the light sources is limited to have the wavelength band specialized for detecting blood glucose.

That is, in general, for infrared spectrometry, light sources for projecting light with continuous wavelength are used. However, in this embodiment, in order to simplify the optical structure and effectively sense and easily analyze signals, infrared light sources which are specialized for blood glucose or which can easily and stably absorb chemical combination in the blood glucose related materials are selected to measure blood glucose.

The plurality of photodiodes by wavelength which are sensitive to infrared light may be also used. Of course, it is also possible to measure the absorption level of blood of the subject to be measured with respect to light with the specific wavelength band in a time-sharing method by using the photodiodes with a single characteristic. When the light source of the first wavelength is turned on by adjustment of four light sources and the photodiodes while the analyzing module is controlled by the program, it is recorded that light sensed by the photodiodes is reflected light or scattered light by the first wavelength light, and it is recorded that light sensed by the photodiodes within a predetermined period of time after second, third and fourth light sources are operated by the predetermined period of time is reflected light or scattered light by light of the corresponding wavelength band.

In order to correct an influence by surrounding light besides the light sources, a method of turning off the four light sources, measuring intensity of light sensed by the photodiode, and obtaining pure intensity of light by wavelength corrected by subtracting the intensity of the sensed light from the intensity sensed when each light source projected light may be also used.

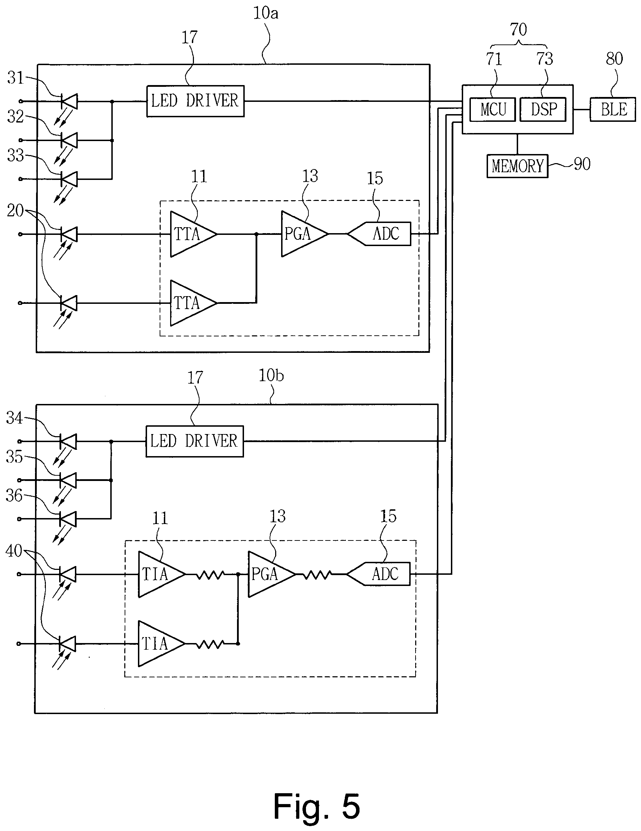

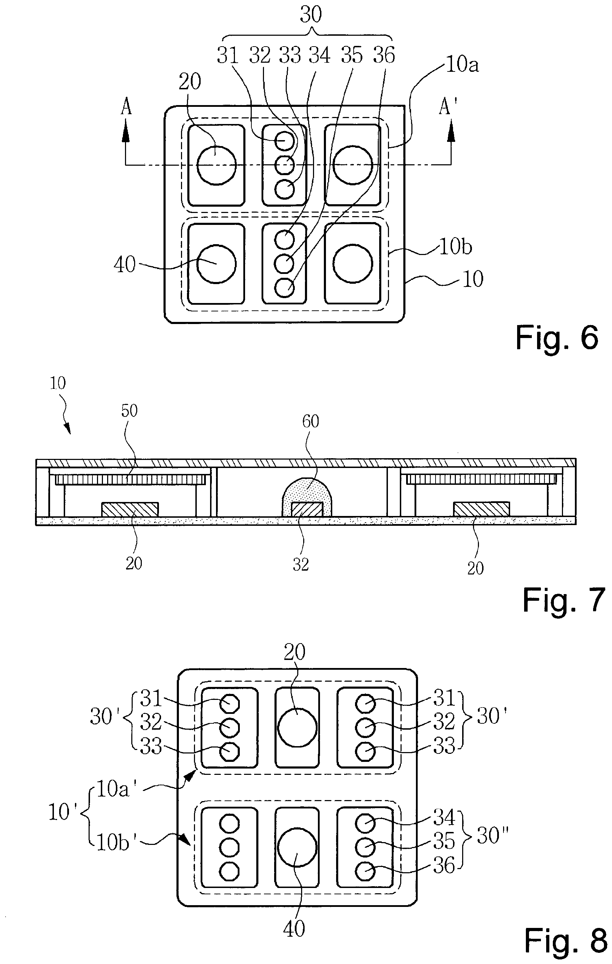

FIG. 5 is a conceptual diagram showing a schematic configuration of a blood glucose monitoring device according to an embodiment of the present invention, FIG. 6 is a bottom side view showing the blood glucose monitoring device based on a photo-plethysmography according to the embodiment of the present invention, and FIG. 7 is a conceptually sectional view taken along the line AA' of FIG. 6.

Referring to FIG. 5, the blood pressure measuring device has two sets of optical sensor assemblies 10 (10a and 10b). Each optical sensor assembly includes three LEDs which are three light sources 30; an LED driver for switching the LEDs or adjusting intensity of the LEDs; two photodiodes as light receiving devices 20 and 40 for receiving scattered or reflected light and generating an electric signal after the light sources project light to a user's body part; two trans-impedance amplifiers (TIA) 11 arranged in a route for acquiring the signal in order to treat the output signal of the photodiodes; a programmable gain amplifier (PGA) 13; and an analog-to-digital converter (ADC) 15. The first optical sensor assembly 10a located at the upper part may be a master, and the second optical sensor assembly 10b located at the lower part may be a slave.

The output signal converted after passing the elements (TIA, PGA and ADC) in each optical sensor assembly is inputted to the common processor 70, and is processed to obtain information for measuring blood glucose from pulse waves. The processor 70 is divided into a digital signal processor (DSP) 73 and a micro-controller unit (MCU) 71. The processor 70 serves to adjust a controller or an LED driver 17 for sending an LED signal to the LEDs, and a memory 90 and a communication unit 80 are connected to the processor.

Not shown in the drawing, but the processor performs various actions, for instance, lighting of the light sources by prefilled program and reference data, output of the photodiodes, calculation of a signal amount by waveform integration by each wavelength range for calculating blood glucose, and obtaining a blood glucose value by substituting to a formula based on the size of the differential signal amount by frequencies related with blood glucose obtained through comparison with the reference data.

Referring to FIG. 6, the optical sensor assemblies 10 are divided into two areas, are located at the upper part and a lower part on the bottom surface, and has three light sources 31, 32 and 33 located at the center of the upper optical sensor assembly 10a, three light sources 34, 35 and 36 located at the center of the lower optical sensor assembly 10b, and the two photodiodes 20 and 40 are mounted at the right and left sides of the light source one by one.

The upper optical sensor assembly 10a includes a green light source 31 with 530 nm wavelength range in a visible ray area, a red light source 32 with 660 nm wavelength range, and a near-infrared light source 33 with 940 nm wavelength range, wherein the light sources are mounted vertically, and the photodiodes 20 disposed at both sides use a photodiode based on a silicon (Si) wafer.

In the lower optical sensor assembly, the near-infrared light sources 34 and 35 with 1200 nm and 1450 nm wavelength ranges and the near-infrared light source 36 with 1700 nm wavelength range are mounted vertically, and the photodiodes 40 disposed at both sides use the photodiodes based on an indium, gallium and arsenic (InGaAs) wafer with excellent sensitivity to the rays with the wavelength ranges.

Of course, it is also possible to form the optical sensor assembly by adding a light source with different near-infrared wavelength range, removing some of the light sources to reduce the number of the light sources, or replacing with a light source with another wavelength range, for instance, the light source with 1450 nm wavelength range may be substituted with a light source with 1100 nm wavelength range. Such a selection is made based on the common standard that there is a light absorption range related with blood glucose to detect the blood glucose level the most distinctly. However, development easiness, such as expenses, element stability, commercialization, and so on, may be considered practically.

When the photodiodes are arranged at both sides of the light source, because sensing ability of the photodiodes may be not satisfied with the sensing ability of the existing commercial photodiodes, the number of the photodiodes is increased to accurately analyze signals.

Moreover, when the blood glucose monitoring device is worn in the form of a wrist band or a watch, there may be a change in wearing position, or the position of the artery may not match the position of the light source and the photodiode. So, in order to solve the problem, the arrangement of the photodiode is made so that even one photodiode can receive the reflected light signal and the scattered light signal.

FIG. 7 is a side sectional view taken along the line of AA of FIG. 6.

A hemispherical convex lens 60 is formed above the LED used as the red light source 32 in order to raise concentration of light projected toward the body part, and a diffractive optical element lens 50 which may be a condensing lens is mounted above the photodiode, which is the photodiode 20, in order to focus light entering from the outside. The photodiode is mounted wider than the LEDs in order to increase a light receiving amount. If the convex lens 60 is mounted above the photodiode, it is difficult to make the blood glucose monitoring device light, small, short and thin due to its height. So, the DOE lens 50 is mounted.

The blood glucose monitoring device of the wrist band type may have additional function part besides an optical noninvasive continuous vital sign monitoring function. For instance, a digital thermometer or a 3D accelerometer may be added in order to monitor other body conditions, physical movement states, or a hurt from a fall. Through the above, the blood glucose monitoring device can analyze correlation with a vital sign trend and monitor an unexpected fluctuation so as to provide the wearer and a medical officer with a warning or am attention signal.

FIG. 8 is a conceptual diagram showing an example of the blood glucose monitoring device having a different arrangement of the light sources and the photodiode.

The optical sensor assembly 10' includes an upper optical sensor assembly 10a' and a lower optical sensor assembly 10b'. Photodiodes 20 and 40 are located in the middle from the two optical sensor assemblies, and three light source modules 30' and 30" respectively having three light sources generating lights with different wavelengths are located at both sides of the photodiodes 20 and 40. The light source modules located at both sides may be the same light source module generating light with the same wavelength, or may be different light source modules with different wavelengths.

Of course, regardless of the case that the different light source modules having different wavelengths are mounted at both sides of the optical sensor assemblies and the case that the light source modules having the same wavelength are mounted at both sides of the optical sensor assemblies, the photodiodes 20 and 40 must differentiate and recognize lights with different wavelengths, and an analyzing module capable of differentiating and recognizing the lights with different wavelengths must be prepared.

In this embodiment, the same light source modules are used at both sides of the optical sensor assemblies, and in this case, lights of the plural wavelengths emitted from the light source modules shine the body part of the subject to be measured, are reflected or scattered, and then, enter the photodiode.

The light sources 30' of the light source modules located at both sides of the photodiode 20 includes a green light source 31 with 530 nm wavelength range in a visible ray region, a red light source 32 with 660 nm wavelength range, and a short near infrared light source 33 with 940 nm wavelength range, which are mounted vertically. The photodiode 20 located in the middle of the light sources is based on a silicon (Si) wafer.

The light sources 30" of the light source modules located at both sides of the photodiode 40 includes near infrared light sources 34 and 35 with 1200 nm and 1450 nm wavelength ranges, and a near infrared light source 36 with 1700 nm wavelength range, which are mounted vertically. The photodiode 40 located in the middle of the light sources is based on an indium, gallium and arsenic (InGaAs) wafer with excellent sensitivity to the rays with the wavelength ranges.

In this instance, it is preferable that detection signals relative to the light sources 31, 32 and 33 of the photodiodes 20 based on the silicon wafer and detection signals relative to the light sources 34, 35 and 36 of the photodiode 40 based on the InGaAs wafer adjust a light receiving area so as to be shown in similar sizes by the analyzing module or adjust sensitivity of the photodiodes. Because light with long wavelength may have small optical energy, it is preferable to adjust the photodiode 40 to be increased in area or sensitivity.

Because the photodiodes sense the sum of lights emitted from the light source modules, the photodiodes receive light twice as much as the photodiodes shown in FIG. 6 that the two photodiodes receive light emitted from one light source module. Therefore, the photodiodes shown in FIG. 8 are less sensitive to external environment and are easy to sense, can obtain an average value to reduce transition by body parts since sensing the sum of reflected lights from two parts of the human body even if there is a delicate difference between the reflected lights and scattered lights by a small difference between body parts. So, the photodiodes shown in FIG. 8 can enhance stability in monitored values.

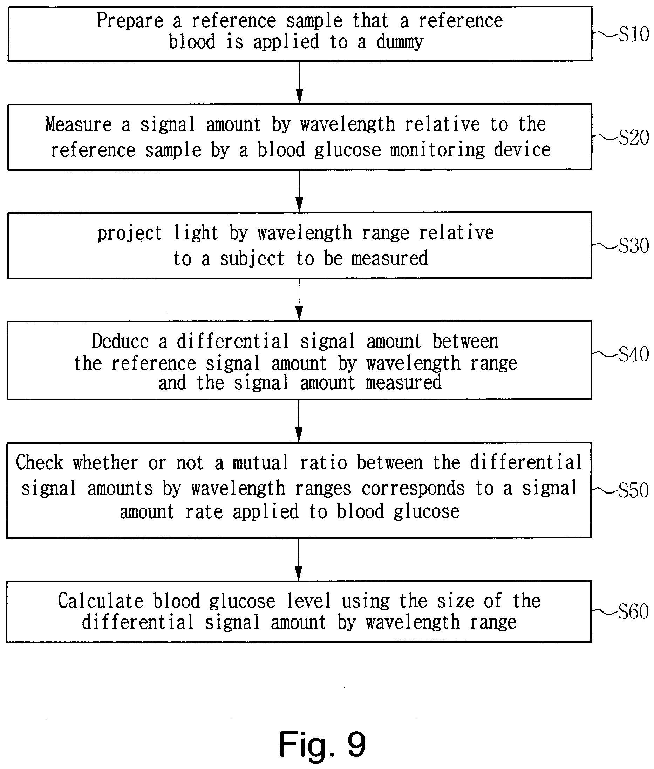

FIG. 9 is a schematic flow chart showing a glucose monitoring method according to the present invention. First, a human body substitute dummy and a reference blood from which blood glucose is removed are prepared, and a reference sample that the reference blood is applied to the dummy is prepared (S10).

Moreover, the blood glucose monitoring device measures a signal amount by wavelength relative to the reference sample to which the reference blood is applied in order to obtain a reference signal amount data (S20). The reference signal amount data may be used as basic material data in the glucose monitoring device in the form of a reference curve by obtaining the reference signal amount relative to all wavelength ranges in substance while densely projecting lights with plural wavelength ranges using a near infrared spectrometer, and may be used as basic material data in the glucose monitoring device in the form of individual reference signal amount set by obtaining the reference signal amount relative to the lights of the light sources with major wavelength ranges of the blood glucose monitoring device. Additionally, the blood glucose monitoring device obtains a plurality of reference curves or individual reference sets by intensity of projected light while varying intensity of projected light or the energy level.

Next the blood glucose monitoring device projects lights of the light sources to the body part of the subject to be measured and obtains the signal amount by wavelength range when the photodiodes receive reflected light and scattered light (S30).

The reference signal amount by corresponding wavelength range included in the basic material data is subtracted from the subject signal amount by wavelength range relative to the body part of the subject to be measured, and then, a differential signal amount by the remaining wavelength range is deduced. (S40).

In this instance, if the reference signal amount in the wavelength range of the light of the reference light source is not equal to the subject signal amount obtained relative to the body part of the subject to be measured using the green light of the reference light source, in order to make the signal amount level equal, the reference curve or the individual reference signal amount set of the basic material data is selected to deduce the differential signal amount by wavelength range using the reference signal amount included in the reference curve or the individual signal amount set.

After the step of deducing the differential signal amount by wavelength, the blood glucose monitoring device checks a mutual ratio between the differential signal amounts by wavelength ranges, and reviews whether or not the rate corresponds to a signal amount rate in wavelength ranges shown by blood glucose (S50). The above step enhance accuracy in blood glucose measurement, and is to prevent disturbance in measurement of blood glucose level by unexpected factors, but is not necessary.

Next, the blood glucose level is calculated using the differential signal amount by wavelength range (S60). If the differential signal amount is big, the blood glucose level is increased. In order to calculate blood glucose level more accurately, correlation between the differential signal amount and the blood glucose level is previously checked so as to be the basic material data embedded in the blood glucose analyzing module, and the blood glucose level can be calculated using the correlation.

While the present invention has been particularly shown and described with reference to exemplary embodiments thereof, it will be understood by those of ordinary skill in the art that the above embodiments of the present invention are all exemplified and the present invention is not limited to the specific embodiments.

Therefore, it will be also understood by those of ordinary skill in the art that various changes and modifications may be made therein without departing from the technical idea and scope of the present invention and such changes and modifications belong to the claims of the present invention.

## Claims

1. A blood glucose monitoring device comprising:
a plurality of light sources for investigating near infrared lights with different wavelengths to a subject to be measured;
light receiving devices for receiving lights reflected and scattered from the subject after being investigated from the plurality of light sources and for converting the received light into an electric signal; and
an analyzing module for analyzing the electric signal of the photodiodes, obtaining a signal amount from a pulse wave type signal relative to each wavelength (in a cyclic integration manner), and calculating blood glucose concentration in blood of the subject using a difference value between the signal amount obtained and a predetermined reference signal amount relative to the reference sample.

2. The blood glucose monitoring device according to claim 1, further comprising:
a green light source for generating green light which is high in absorption level relative to a base component and is little related with blood glucose as a reference light source, besides the plurality of light sources.

3. The blood glucose monitoring device according to claim 1 or 2, wherein the analyzing module uses a mutual ratio of the difference value obtain by each wavelength range of the plurality of light sources as a verification means in order to enhance accuracy in calculation of a blood glucose numerical value.

4. The blood glucose monitoring device according to claim 1, wherein the body part is the wrist, and the light receiving devices and the light sources are mounted on a body facing surface of a band worn on the wrist.

5. The blood glucose monitoring device according to claim 1, wherein the plurality of light sources include at least two light sources among a short near infrared light source with 940nm wavelength range, a near infrared light source with 1200nm wavelength range, a near infrared light source with 1450nm wavelength range, and a near infrared light source with 1700nm wavelength range.

6. The blood glucose monitoring device according to claim 1, comprising:
at least one optical sensor assembly having two light source modules having light sources mounted at both sides of the light receiving device.

7. The blood glucose monitoring device according to claim 1, further comprising:
at least one optical sensor assembly in which a light source module having light sources mounted between the light receiving devices.

8. A blood glucose monitoring method for measuring blood glucose level using the blood glucose monitoring device of claim 1, comprising the steps of:
preparing a reference sample by applying a reference blood that blood glucose is removed from blood components to a human body substitute dummy;
acquiring signal amounts by wavelength ranges relative to the reference sample by the blood glucose monitoring device or other accurate blood glucose monitoring device;
acquiring signal amounts by wavelength ranges relative to a body part of a subject to be measured by the blood glucose monitoring device;
subtracting the signal amounts by wavelength ranges relative to the reference sample from the signal amounts by wavelength ranges relative to the body part of the subject to be measured, and deducing differential signal amounts by wavelength ranges; and
calculating the blood glucose level using the differential signal amounts by wavelength ranges.

9. The blood glucose monitoring method according to claim 8, wherein the blood glucose monitoring device further includes a light source with green light wavelength range (530nm) in a visible light region as the reference light source in order to measure signal amounts besides the plurality of light sources, in consideration of transition by the base component,
wherein a step of adjusting in such a way that the signal amounts relative to the reference sample becomes equal to the signal amounts measured relative to the body part of the subject to be measured with respect to the projected light of the reference light source, and adjusting the signal amounts by each wavelength range relative to the body part of the subject to be measured is further included, and
wherein the step of subtracting the signal amounts by wavelength range relative to the reference sample from the signal amounts by wavelength range relative to the body part of the subject to be measured, and deducing differential signal amounts by wavelength range deduces the differential signal amounts by wavelength range using the adjusted signal amounts by wavelength range.

10. The blood glucose monitoring method according to claim 8 or 9, further comprising the step of:
inspecting the mutual ratio between the differential signal amounts by wavelength ranges and checking whether or not the mutual ratio accords with the signal amount rate in the wavelength ranges relative to the blood glucose after the step of deducing the differential signal amounts by the remaining wavelength range.

11. The blood glucose monitoring method according to claim 8 or 9, wherein the step of preparing a reference sample by applying a reference blood that blood glucose is removed from blood components to a human body substitute dummy and the step of acquiring signal amounts by wavelength ranges relative to the reference sample by the blood glucose monitoring device or other accurate blood glucose monitoring device are previously carried out before measuring the subject to be measured, so that the reference data is stored in the analyzing module and is prepared in such a way as to be drawn out and carry out comparison in case of necessity.
